# FASCICULE DE BREVET EUROPEEN

(11) **EP 4 572 827 B1**
(45) Date de publication et mention de la délivrance du brevet: **08.04.2026**
(21) Numéro de dépôt: 23768327.1
(22) Date de dépôt: 16.08.2023
(51) Int. Cl.: A61M 15/00

(54) **INHALATEUR DE POUDRE SÈCHE**
TROCKENPULVERINHALATOR
DRY-POWDER INHALER

(30) Priorité: 18.08.2022 FR 2208366
(43) Date de publication de la demande: 25.06.2025
(73) Titulaire: APTAR France SAS, 27110 Le Neubourg (FR)
(72) Inventeur: POULLAIN, Franck, 27400 LA HAYE MALHERBE (FR)
(74) Mandataire: CAPRI
(86) Numéro de dépôt international: PCT/FR2023/051274
(87) Numéro de publication internationale: WO 2024/038236

(56) Documents cités:
- WO-A1-2008/001132
- WO-A1-2013/008037
- WO-A2-2012/163704
- WO-A2-98/26828

## Description

La présente invention concerne un inhalateur de poudre sèche.

Les inhalateurs de poudre sont bien connus dans l'état de la technique. Il en existe différentes sortes.

Un premier type d'inhalateur multidoses contient un réservoir recevant une multitude de doses de poudre, l'inhalateur étant pourvu de moyens de dosage permettant à chaque actionnement de séparer une dose de cette poudre du réservoir pour l'amener dans un conduit d'expulsion afin d'être distribué à l'utilisateur. Ces dispositifs nécessitent des moyens de dosage complexes et donc coûteux, et la précision et la reproductibilité du dosage ne sont pas garanties. De plus, il y a des risques de contamination de la poudre disposée dans le réservoir au fur et à mesure de l'utilisation de l'inhalateur.

Un autre type d'inhalateur multidoses consiste à prévoir plusieurs réservoirs individuels contenant chacun une dose de poudre, puis d'ouvrir un de ces réservoirs à chaque actionnement de l'inhalateur. Cette mise en œuvre assure une meilleure étanchéité de la poudre, puisque chaque dose de poudre n'est exposée à l'atmosphère qu'au moment de son expulsion. Pour réaliser ces ensembles de réservoirs individuels, diverses variantes ont déjà été proposées, telle que des bandes ou disques de blisters. Concernant l'ouverture des réservoirs individuels, il a été proposé de peler ou décoller la couche de fermeture des blisters. Ceci présente l'inconvénient d'une maîtrise difficile des forces à appliquer pour garantir une ouverture totale sans risquer d'ouvrir le réservoir suivant, particulièrement si les moyens d'ouverture doivent être actionnés par l'inhalation. Une autre solution est de percer la membrane de fermeture d'un blister à chaque actionnement, ce qui requiert des moyens de perçage complexes et donc coûteux.

Les inhalateurs multidoses décrits ci-dessus présentent de plus le problème de ne pas garantir une efficacité de la distribution optimale, avec une partie importante de la dose qui pénètre effectivement dans les poumons de l'utilisateur pour avoir un effet thérapeutique bénéfique.

Pour réaliser des dispositifs moins complexes et donc moins coûteux, il a été proposé des inhalateurs unidoses comportant un seul réservoir individuel, tels qu'un blister ou une capsule, qui est à charger dans l'inhalateur juste avant l'utilisation de celui-ci. L'avantage de ces dispositifs unidoses est qu'il n'est pas nécessaire de stocker l'ensemble des doses à l'intérieur de l'appareil, de sorte que celui-ci peut être de dimension réduite. Par contre, l'utilisation est plus compliquée pour l'utilisateur, puisque celui-ci est obligé de charger un blister ou une capsule dans l'inhalateur avant chaque utilisation. De plus, d'autres inconvénients spécifiques à ces inhalateurs unidoses existent. Ainsi, un blister ou une capsule ne permet pas la distribution de grandes doses, typiquement supérieures à 100 mg. De plus, l'ouverture, notamment des capsules ou blisters à percer, impose l'utilisation de moyens de perçage complexes, ainsi qu'un risque de résidus de membrane percée dans la poudre distribuée. Par ailleurs, les performances de vidage du réservoir ne sont pas toujours optimales, une partie de la poudre pouvant rester à l'intérieur du réservoir lors de l'inhalation.

Pour tenter de pallier à ces inconvénients, il a été proposé dans le document WO9826828 un inhalateur unidose comportant un réservoir cylindrique pourvu d'une ouverture latérale, disposé dans une chambre cylindrique de plus grand diamètre, de sorte que lors de l'inhalation, le réservoir se déplace dans la chambre selon un mouvement orbital, ce qui provoque l'expulsion de la poudre. Cette mise en œuvre permet effectivement d'améliorer l'efficacité de la distribution, avec un vidage sensiblement total du réservoir et une partie plus importante de la dose qui pénètre effectivement dans les poumons. Néanmoins, un inconvénient de ce dispositif est que l'ouverture latérale du réservoir doit être ouverte avant la mise en place du réservoir dans le dispositif, ce qui présente un risque de perte de poudre lors de cette mise en place, ou de contamination de celle-ci, notamment si le dispositif n'est pas utilisé rapidement après la mise en place du réservoir. De plus, en cas de réutilisation de l'inhalateur avec un autre réservoir, la partie formant embout buccal risque d'être polluée par l'utilisation précédente, en particulier la grille de protection prévue en amont de l'orifice de distribution de l'inhalateur.

Les documents WO2013008037A1, WO2012163704A2, WO2008001132A1 et WO9826828A2 décrivent d'autres dispositifs de l'état de la technique.

La présente invention a pour but de fournir un inhalateur de poudre qui ne reproduit pas les inconvénients susmentionnés.

En particulier, la présente invention a pour but de fournir un tel inhalateur qui puisse aisément être réutilisé avec plusieurs réservoirs, sans risques de pollution ou de contamination de l'inhalateur.

La présente invention a également pour but de fournir un tel inhalateur qui limite voire empêche les risques de contamination et/ou de pollution de la poudre contenue dans le réservoir.

La présente invention a également pour but de fournir un tel inhalateur qui réduit la complexité d'utilisation pour l'utilisateur et qui garantit une meilleure sécurité d'utilisation.

La présente invention a aussi pour but de fournir un tel inhalateur qui soit simple et peu coûteux à fabriquer et à assembler.

La présente invention a donc pour objet un inhalateur de poudre sèche comportant:
- un corps contenant une chambre et un manchon de réception,
- une unité réservoir destinée à être insérée de manière amovible dans ledit manchon de réception, ladite unité réservoir comportant:
   (i) un réservoir contenant une dose de poudre sèche à inhaler et comportant un orifice latéral de sortie, ledit réservoir étant déplaçable dans ledit inhalateur entre une position non chargée, dans laquelle il est disposé dans ledit manchon de réception, et une position chargée, dans laquelle il est entièrement disposé dans ladite chambre,
   (ii) une bague de fermeture déplaçable par rapport audit réservoir entre une position de fermeture dudit orifice latéral de sortie et une position d'ouverture dudit orifice latéral de sortie, ladite bague de fermeture étant en position de fermeture quand ledit réservoir est en position non chargée, et étant en position d'ouverture quand ledit réservoir est en position chargée,
   (iii) un organe d'actionnement formant embout buccal, pour déplacer ledit réservoir de sa position non chargée vers sa position chargée, ledit organe d'actionnement définissant un orifice de distribution dudit inhalateur.

Avantageusement, ledit organe d'actionnement comporte une grille en amont dudit orifice de distribution.

Avantageusement, ladite chambre est disposée entre une entrée d'air d'inhalation et ledit manchon de réception.

Avantageusement, le diamètre dudit réservoir est inférieur au diamètre de ladite chambre.

Avantageusement, des canaux tangentiels amènent un flux d'air d'inhalation de manière tangentielle dans ladite chambre, ce qui génère un déplacement orbital dudit réservoir dans ladite chambre.

Avantageusement, ledit réservoir contient une dose de poudre supérieure à 50 mg, notamment supérieure à 100 mg.

Avantageusement, le volume occupé par ledit réservoir dans ladite chambre en position chargée est supérieur à 50% du volume de ladite chambre.

Avantageusement, le diamètre radial dudit réservoir est supérieur à sa hauteur axiale.

Avantageusement, ledit manchon de réception comporte sur sa surface externe un repère visuel, par exemple une ligne colorée, visible par l'utilisateur lorsque ledit réservoir est en position non chargée, et invisible lorsque ledit réservoir est en position chargée.

Ces caractéristiques et avantages et d'autres de la présente invention apparaîtront plus clairement au cours de la description détaillée suivante, faite en référence aux dessins joints, donnés à titre d'exemples non limitatifs, sur lesquels
la figure 1 est une vue schématique en section transversale d'un inhalateur selon un mode de réalisation avantageux, avant insertion du réservoir dans l'inhalateur,
la figure 2 est une vue similaire à celle de la figure 1, après assemblage du réservoir mais en position non chargée,
la figure 3 est une vue similaire à celle de la figure 2, en position chargée,
la figure 4 est une vue schématique de dessous du réservoir dans la chambre, en position chargée, et
la figure 5 est une vue schématique de dessus de l'embout buccal, en position chargée.

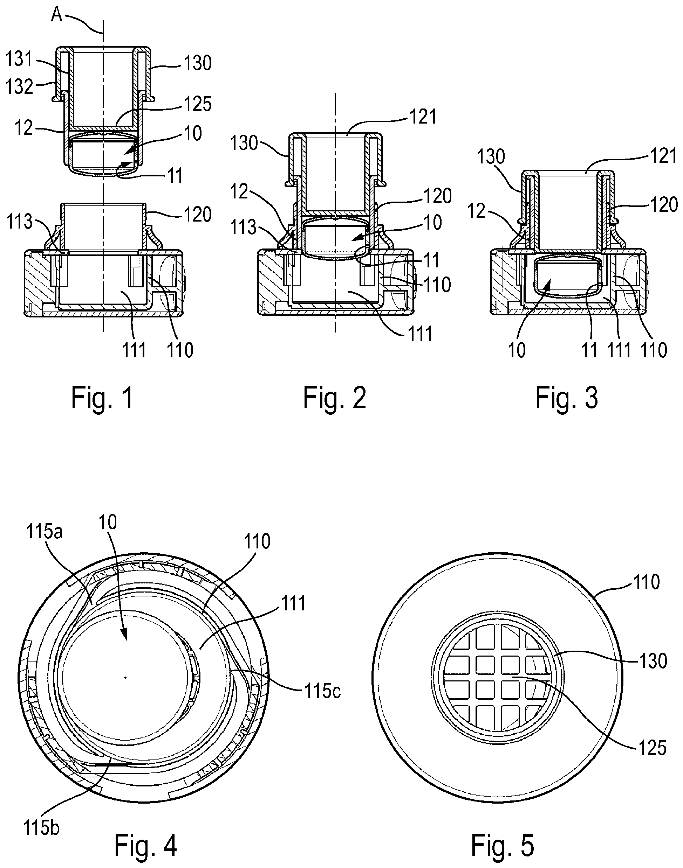

Dans la description ci-après, les termes "supérieur", "inférieur" et "latéral" se réfèrent à la position droite du dispositif représenté sur les figures 1 à 3. Les termes "axial" et "radial" se réfèrent à l'axe central longitudinal A du dispositif, représenté sur la figure 1.

Dans le mode de réalisation représenté sur les dessins, l'inhalateur comporte un corps 110 contenant une chambre 111 et un manchon de réception 120 adapté à recevoir une unité réservoir amovible. De préférence, la chambre 111 est disposée entre une entrée d'air d'inhalation et ledit manchon de réception 120, de sorte que lorsque l'utilisateur inhale, le flux d'air va traverser ladite chambre 111.

L'unité réservoir comporte un réservoir 10, une bague de fermeture 12 et un organe d'actionnement 130, formant embout buccal et définissant l'orifice de distribution 121.

Le réservoir 10 contient une dose de poudre sèche à inhaler et comporte un orifice latéral de sortie 11. Le réservoir 10 est adapté à être disposé de manière axialement déplaçable dans l'inhalateur entre une position non chargée, représentée sur la figure 2, et une position chargée, représentée sur la figure 3. Dans la position non chargée, le réservoir 10 est au moins partiellement disposé hors de ladite chambre 111, en étant positionné dans le manchon de réception 120. Dans la position chargée, il est entièrement disposé dans ladite chambre 111.

Le réservoir 10 est inséré dans la bague de fermeture 12 adaptée à fermer ledit orifice latéral de sortie 11. Cette insertion se fait de préférence par un emmanchement à force, de sorte que le réservoir 10 est retenu par frottements dans ladite bague de fermeture 12. Cette bague de fermeture 12 protège donc la poudre contenue dans le réservoir 10 jusqu'à sa distribution par l'actionnement de l'inhalateur. La bague de fermeture 12 est déplaçable et/ou déformable par rapport au réservoir 10 entre des positions de fermeture et d'ouverture de l'orifice latéral de sortie 11, ladite bague de fermeture 12 étant en position de fermeture quand ledit réservoir 10 est en position non chargée, et étant en position d'ouverture quand ledit réservoir 10 est en position chargée.

La bague de fermeture 12 est fixée de manière axialement déplaçable dans l'organe d'actionnement 130, qui sert pour déplacer ledit réservoir 10 de sa position non chargée vers sa position chargée. Cet organe d'actionnement 130 est déplaçable axialement par rapport au corps 110 pour pousser le réservoir 10 de sa position non chargée vers sa position chargée. Avantageusement, l'organe d'actionnement 130 comporte deux parois coaxiales 131, 132 entre lesquelles ladite bague de fermeture 12 peut coulisser entre ses positions de fermeture et d'ouverture. Avantageusement, comme visible sur la figure 5, l'organe d'actionnement 130 comporte une grille 125 en amont de l'orifice de distribution 121, au contact du réservoir 10.

Ainsi, avant insertion de l'unité réservoir dans l'inhalateur, le réservoir 10 est disposé dans la bague de fermeture 12 qui est en position de fermeture.

Lorsque l'unité réservoir est inséré dans l'inhalateur, le bord inférieur de la bague de fermeture 12 vient en butée contre une projection radiale 113 du corps 110. Le réservoir 10 est alors en position non chargée, avec toujours l'orifice latéral de sortie 11 obturé par la bague de fermeture 12.

Lorsque l'utilisateur souhaite utiliser l'inhalateur, il déplace l'organe d'actionnement 130 axialement vers le bas par rapport au manchon de réception 112 pour pousser le réservoir 10 de sa position non chargée vers sa position chargée. La bague de fermeture 12 étant axialement bloquée par la projection radiale 113, le réservoir 10 est extrait de ladite bague de fermeture 12 pour venir dans la chambre 111, en position chargée, avec l'orifice latéral de sortie 11 ouvert.

Avantageusement, le manchon de réception 120 peut comporter sur sa surface externe un repère visuel, par exemple une ligne colorée, visible par l'utilisateur lorsque le réservoir 10 est en position non chargée, et qui n'est plus visible lorsque le réservoir 10 est en position chargée. Ceci permet d'informer l'utilisateur que le déplacement de l'organe d'actionnement 130 a été correctement réalisé. Ainsi, tant que le repère visuel reste visible, l'utilisateur sait que le réservoir 10 n'est pas encore en position chargée, et qu'il ne doit pas inhaler, et ce n'est que lorsque le repère visuel n'est plus visible, en étant masqué par l'organe d'actionnement 130, que l'utilisateur va inhaler.

Le diamètre du réservoir 10 est inférieur au diamètre de ladite chambre 111, de sorte qu'en position chargée, le réservoir 10 peut se déplacer dans la chambre 111. Avantageusement, des canaux tangentiels 115a, 115b, 115c amène ledit flux d'air d'inhalation de manière tangentielle dans la chambre 111, ce qui va provoquer un déplacement orbital du réservoir 10 dans la chambre 111, le réservoir 10 tournant sur lui-même tout en tournant autour de la périphérie de la chambre 111 le long de la paroi latérale de celle-ci. Ce mouvement va permettre l'expulsion de la poudre contenue dans le réservoir 10, qui est entrainée par le flux d'air d'inhalation vers l'orifice de distribution.

Avantageusement, le réservoir 10 peut contenir une dose de poudre supérieure à 50 mg, notamment supérieure à 100 mg. Eventuellement, des doses très importantes peuvent être envisagées, notamment supérieures à 500 mg.

Avantageusement, le volume occupé par le réservoir 10 dans la chambre 111 en position chargée est supérieur à 50% du volume de la chambre 111.

Avantageusement, le diamètre radial du réservoir 10 est supérieur à sa hauteur axiale.

Avantageusement, le réservoir 10 est formé d'une partie supérieure de réservoir et d'une partie inférieure de réservoir, qui sont assemblées après remplissage avec la dose de poudre. Avantageusement, c'est la partie inférieure de réservoir qui comporte l'orifice latéral de sortie 11.

Un avantage de l'unité réservoir est qu'elle n'est pas symétrique, ce qui forme un détrompage pour l'insertion du réservoir 10 dans le corps 110 de l'inhalateur, cette insertion n'étant possible que dans une seule orientation du réservoir 10. Ceci est notamment avantageux lorsque la forme extérieure du réservoir 10 est sensiblement symétrique mais que l'orifice latéral de sortie 11 n'est pas disposée au centre axial du réservoir 10. En effet, dans ce cas, il est souhaitable d'orienter correctement le réservoir 10 dans l'inhalateur pour un fonctionnement optimal, et la forme asymétrique de l'unité réservoir permet de remplir cette fonction.

Le dispositif de l'invention est simple et efficace. Il est constitué d'un faible nombre de pièces, il est donc peu coûteux à fabriquer et à assembler, et fiable d'utilisation. Il permet une distribution optimale de la poudre de par le mouvement orbital du réservoir lors de l'actionnement, tout en garantissant l'intégrité de la poudre jusqu'à son utilisation.

Il est à noter que l'inhalateur peut être rechargé très facilement, en remplaçant l'unité réservoir avec un réservoir vide par une unité réservoir avec un réservoir plein. Ainsi, la grille 125 et l'organe d'actionnement 130 formant l'embout buccal sont remplacés après chaque utilisation de l'inhalateur, ensemble avec le réservoir, de sorte qu'il n'y a aucun risque de pollution ou de contamination de l'inhalateur suite à la précédente utilisation.

Diverses modifications sont également possibles pour un homme du métier sans sortir du cadre de la présente invention tel que défini par les revendications annexées.

## Revendications

1. Inhalateur de poudre sèche comportant:
- un corps (110) contenant une chambre (111) et un manchon de réception (120),
- une unité réservoir destinée à être insérée de manière amovible dans ledit manchon de réception (120), **caractérisé en ce que** ladite unité réservoir comporte:
(i) un réservoir (10) contenant une dose de poudre sèche à inhaler et comportant un orifice latéral de sortie (11), ledit réservoir (10) étant déplaçable dans ledit inhalateur entre une position non chargée, dans laquelle il est disposé dans ledit manchon de réception (120), et une position chargée, dans laquelle il est entièrement disposé dans ladite chambre (111),
(ii) une bague de fermeture (12) déplaçable par rapport audit réservoir (10) entre une position de fermeture dudit orifice latéral de sortie (11) et une position d'ouverture dudit orifice latéral de sortie (11), ladite bague de fermeture (12) étant en position de fermeture quand ledit réservoir (10) est en position non chargée, et étant en position d'ouverture quand ledit réservoir (10) est en position chargée,
(iii) un organe d'actionnement (130) formant embout buccal, pour déplacer ledit réservoir (10) de sa position non chargée vers sa position chargée, ledit organe d'actionnement définissant un orifice de distribution dudit inhalateur.

2. Inhalateur selon la revendication 1, dans lequel ledit organe d'actionnement (130) comporte une grille (125) en amont dudit orifice de distribution (121).

3. Inhalateur selon la revendication 1 ou 2, dans lequel ladite chambre (111) est disposée entre une entrée d'air d'inhalation et ledit manchon de réception (120).

4. Inhalateur selon l'une quelconque des revendications précédentes, dans lequel le diamètre dudit réservoir (10) est inférieur au diamètre de ladite chambre (111).

5. Inhalateur selon l'une quelconque des revendications précédentes, dans lequel des canaux tangentiels (115a, 115b, 115c) amènent un flux d'air d'inhalation de manière tangentielle dans ladite chambre (111), ce qui génère un déplacement orbital dudit réservoir (10) dans ladite chambre (111).

6. Inhalateur selon l'une quelconque des revendications précédentes, dans lequel ledit réservoir (10) contient une dose de poudre supérieure à 50 mg, notamment supérieure à 100 mg.

7. Inhalateur selon l'une quelconque des revendications précédentes, dans lequel le volume occupé par ledit réservoir (10) dans ladite chambre (111) en position chargée est supérieur à 50% du volume de ladite chambre (111).

8. Inhalateur selon l'une quelconque des revendications précédentes, dans lequel le diamètre radial dudit réservoir (10) est supérieur à sa hauteur axiale.

9. Inhalateur selon l'une quelconque des revendications précédentes, dans lequel ledit manchon de réception (120) comporte sur sa surface externe un repère visuel, par exemple une ligne colorée, visible par l'utilisateur lorsque ledit réservoir (10) est en position non chargée, et invisible lorsque ledit réservoir (10) est en position chargée.

## Patentansprüche

1. Trockenpulverinhalator mit:
- einem Körper (110), der eine Kammer (111) und eine Aufnahmehülse (120) enthält,
- eine Reservoir-Einheit, die dazu bestimmt ist, abnehmbar in die Aufnahmehülse (120) eingesetzt zu werden,
**dadurch gekennzeichnet, dass** die Reservoir-Einheit aufweist:
(i) ein Reservoir (10), das eine Dosis eines zu inhalierenden Trockenpulvers enthält und einen seitlichen Auslass (11) aufweist, wobei das Reservoir (10) in dem Inhalator zwischen einer nicht aufgeladenen Position, in der es in der Aufnahmehülse (120) angeordnet ist, und einer aufgeladenen Position, in der es vollständig in der Kammer (111) angeordnet ist, verschiebbar ist,
(ii) einen Verschlussring (12), der relativ zu dem Reservoir (10) zwischen einer Verschlussposition des seitlichen Auslasses (11) und einer Öffnungsposition des seitlichen Auslasses (11) verschiebbar ist, wobei der Verschlussring (12) sich in der Verschlussposition befindet, wenn sich das Reservoir (10) in der nicht aufgeladenen Position befindet, und sich in der Öffnungsposition befindet, wenn sich das Reservoir (10) in der aufgeladenen Position befindet,
(iii) ein Betätigungselement (130), das ein Mundstück bildet, um das Reservoir (10) von seiner nicht aufgeladenen Position in seine aufgeladene Position zu verschieben, wobei das Betätigungselement einen Auslass des Inhalators definiert.

2. Inhalator nach Anspruch 1, wobei das Betätigungselement (130) ein Gitter (125) stromaufwärts des Auslasses (121) aufweist.

3. Inhalator nach Anspruch 1 oder 2, wobei die Kammer (111) zwischen einem Lufteinlass für die Inhalation und der Aufnahmehülse (120) angeordnet ist.

4. Inhalator nach einem der vorhergehenden Ansprüche, wobei der Durchmesser des Reservoirs (10) kleiner ist als der Durchmesser der Kammer (111).

5. Inhalator nach einem der vorhergehenden Ansprüche, wobei tangentiale Kanäle (115a, 115b, 115c) einen Inhalationsluftstrom tangential in die Kammer (111) einbringen, wodurch eine orbitale Bewegung des Reservoirs (10) in der Kammer (111) erzeugt wird.

6. Inhalator nach einem der vorhergehenden Ansprüche, wobei das Reservoir (10) eine Pulverdosis von mehr als 50 mg, insbesondere von mehr als 100 mg, enthält.

7. Inhalator nach einem der vorhergehenden Ansprüche, wobei das von dem Reservoir (10) in der aufgeladenen Position in der Kammer (111) eingenommene Volumen größer als 50 % des Volumens der Kammer (111) ist.

8. Inhalator nach einem der vorhergehenden Ansprüche, wobei der radiale Durchmesser des Reservoirs (10) größer ist als seine axiale Höhe.

9. Inhalator nach einem der vorhergehenden Ansprüche, wobei die Aufnahmehülse (120) auf ihrer äußeren Oberfläche eine visuelle Markierung, beispielsweise eine farbige Linie, aufweist, die für den Benutzer sichtbar ist, wenn sich das Reservoir (10) in der nicht aufgeladenen Position befindet, und unsichtbar ist, wenn sich das Reservoir (10) in der aufgeladenen Position befindet.

## Claims

1. A dry-powder inhaler comprising:
- a body (110) containing a chamber (111) and a receiving sleeve (120),
- a reservoir unit intended to be removably inserted into said receiving sleeve (120), **characterized in that** said reservoir unit comprises:
(i) a reservoir (10) containing a dose of dry powder to be inhaled and comprising a lateral outlet opening (11), said reservoir (10) being movable in said inhaler between a non-loaded position in which it is disposed in said receiving sleeve (120), and a loaded position in which it is disposed entirely in said chamber (111),
(ii) a closure ring (12) movable relative to said reservoir (10) between a closed position of said lateral outlet opening (11) and an open position of said lateral outlet opening (11), said closure ring (12) being in a closed position when said reservoir (10) is in the non-loaded position, and being in an open position when said reservoir (10) is in the loaded position,
(iii) an actuator member (130) forming a mouthpiece, for moving said reservoir (10) from its non-loaded position to its loaded position, said actuator member defining a dispensing orifice of said inhaler.

2. The inhaler according to claim 1, wherein said actuator member (130) comprises a grid (125) upstream from said dispensing orifice (121).

3. The inhaler according to claim 1 or claim 2, wherein said chamber (111) is disposed between an inhalation air inlet and said receiving sleeve (120).

4. The inhaler as claimed in any one of the preceding claims, wherein the diameter of said reservoir (10) is less than the diameter of said chamber (111).

5. The inhaler as claimed in any one of the preceding claims, wherein tangential channels (115a, 115b, 115c) bring a flow of inhalation air into said chamber (111) in a tangential manner, which generates an orbital displacement of said reservoir (10) in said chamber (111).

6. The inhaler as claimed in any one of the preceding claims, wherein said reservoir (10) contains a dose of powder greater than 50 mg, in particular greater than 100 mg.

7. The inhaler as claimed in any one of the preceding claims, in which the volume occupied by said reservoir (10) in said chamber (111) in the loaded position is greater than 50% of the volume of said chamber (111).

8. The inhaler as claimed in any one of the preceding claims, in which the radial diameter of said reservoir (10) is greater than its axial height.

9. The inhaler as claimed in any one of the preceding claims, wherein said receiving sleeve (120) comprises on its outer surface a visual mark, for example a coloured line that can be seen by the user when said reservoir (10) is in the non-loaded position, and that cannot be seen when said reservoir (10) is in the loaded position.
